# EUROPEAN PATENT APPLICATION

(11) **EP 2 604 228 A1**
(43) Date of publication of application: **19.06.2013**
(21) Application number: 11816476.3
(22) Date of filing: 10.08.2011
(51) Int. Cl.: A61F 2/82, A61L 31/00

(54) **STENT**

(30) Priority: 12.08.2010 JP 2010181172
(71) Applicant: Optnics Precision Co., Ltd., Ashikaga-shi, Tochigi 326-0037 (JP)
(72) Inventor: KINUTA, Seichin, Ashikaga-shi Tochigi 326-0037 (JP); KAWABATA, Takashi, Saitama-shi Saitama 338-0001 (JP)
(74) Representative: Kensett, John Hinton
(86) International application number: PCT/JP2011/068314
(87) International publication number: WO 2012/020812

(57) **Abstract**

A stent that is extremely useful in practice, with excellent proof stress (elastic limit stress).

The stent (1) is in a tubular shape that is expandable in a radial direction, the stent being placed in a tubular vessel of a living body. The stent (1) is formed of a main stent element (2) that is formed at an imaginary cylinder surface. The main stent element (2) is fabricated of an alloy with a proof stress of from 500 to 2700 MPa, or is fabricated of an electroformed alloy with a repetition fatigue strength at least 1.5 times the strength of a nickel member or a nickel/cobalt alloy member, which is an alloy containing any of gold, silver, copper, nickel, cobalt and palladium. Therefore, the stent (1) may be easily and uniformly expanded in the radial direction, and is not easily crushed in the radial direction after being expanded.

## Description

### TECHNICAL FIELD

The present invention relates to a stent that is placed in a constriction portion or the like in a tubular vessel in the body, such as a blood vessel, the gallbladder, the esophagus, the intestines, the ureter or the like.

### BACKGROUND ART

A stent is, for example, used with a balloon catheter. When there is a constriction in a tubular vessel of the body such as a blood vessel or the like, the constriction portion is expanded by the balloon catheter, after which the stent is left in place. The stent supports interior walls of the tubular vessel from thereinside, and is used for preventing a restenosis of the vessel. When the stent is being inserted, the stent is installed in a reduced-diameter state at the outer side of a balloon that is in a deflated state, and is inserted into the body tubular vessel together with the balloon. When the balloon has been disposed in the constriction portion, the stent is expanded by the balloon being inflated, the constriction portion is expanded, the stent is left in place in an expanded state, and the balloon catheter alone is taken out.

The characteristics required of a stent that is to be used in this manner include excellent insertability into the body tubular vessel, longitudinal flexibility in the reduced-diameter state, the ability to easily and uniformly expand in the radial direction and, after expansion, not being easily crushed in the radial direction. Such stents are proposed in, for example, Patent Document 1 and Patent Document 2.

### Related Art References

### Patent References

Patent Document 1: Japanese Patent Application Laid-Open (JP-A) No. 2007-267844
Patent Document 2: JP-A No. 2000-8187

### SUMMARY OF INVENTION

### TECHNICAL PROBLEM

However, the stent recited in the above-mentioned Patent Document 1 is unreliable in practice, because a bottom limit on proof stress is low at 300 MPa, and no upper limit is disclosed. Furthermore, because the stent of Patent Document 1 is fabricated by laser machining, the stent may be affected by heating, and annealing or brittleness due to thermal deformation may occur. In addition, steps of deburring, polishing and the like are necessary. Thus, the fabrication process is complicated, among other issues.

The stent recited in the above-mentioned Patent Document 2 is fabricated by electroforming, and the fabrication is easier than laser machining. However, gold, which is given as an example of the metal that is electroformed, by itself has inferior proof stress. Therefore, there are problems with using this stent in practice, among other issues.

The present invention is addressed to the problem described above, may provide successful deposition of various alloys by electrolytic deposition in a metal ion solution, and may provide an alloy electroformed member that provides excellent mechanical characteristics and corrosion resistance in contrast to usual raw materials, that is, metallic materials that are obtained by dissolution and rolling, which are associated with high temperatures. Hence, an object of the present invention is to provide a stent that is extremely useful in practice, being provided with an alloy that is excellent in proof stress (elastic limit stress), that has high fatigue strength with respect to the beating of the heart, and that does not elute metal ions into any body fluid or an electrolyte.

### SOLUTION TO PROBLEM

A stent according to a first aspect of the present invention is a tubular stent that is expandable in a radial direction, the stent being placed in a tubular vessel of a living body, and including a main stent element that is formed at an imaginary cylinder surface, the main stent element being fabricated of an alloy with a proof stress of from 500 to 2700 MPa.
Thus, the lower limit on proof stress of the stent element is 500 MPa, which is greater than the 300 MPa of the stent recited in Patent Document 1, and the upper limit is 2700 MPa. Therefore, even a stent with a very thin wall thickness may withstand external pressures, and when the stent is disposed in a blood vessel, cross-sectional area of the blood vessel is not sacrificed. In addition, the stent may be easily and uniformly expanded in the radial direction, and the stent is not easily crushed in the radial direction after being expanded.

A stent according to a second aspect of the present invention is a tubular stent that is expandable in a radial direction, the stent being placed in a tubular vessel of a living body, and including a main stent element that is formed at an imaginary cylinder surface, the main stent element being fabricated of an alloy with a higher fatigue strength than nickel and nickel/cobalt alloys.

In a stent according to a third aspect of the present invention, the fatigue strength of the alloy of the main stent element is at least 1.5 times the strength of nickel and nickel/cobalt alloys.
Thus, the stent is provided with a higher fatigue strength than a related art stent fabricated by electroforming of nickel or a nickel/cobalt alloy, and is preferably provided with a fatigue strength at least 1.5 times that of the related art stent.

In a stent according to a fourth aspect of the present invention, the main stent element is an electroformed alloy including one of gold, silver, copper, nickel, cobalt or palladium.
Because the stent is produced by electroforming a material with a very high fatigue strength, this is particularly favorable for a stent in a location that is continuously subjected to vibrations. In addition, the stent may be easily and uniformly expanded in the radial direction, and not easily crushed in the radial direction after expansion.

A stent according to a fifth aspect of the present invention is fabricated by electroforming using the alloy including one of gold, silver, copper, nickel, cobalt or palladium, to which is added a compound selected from sulfites, chlorides, ammonia complexes, cyan complexes, amine complexes, sulfamine complexes, hypophosphites, pyrophosphates, tartrates or EDTA.
Because of the use of a selected compound as an additive, a stent with high proof stress and fatigue strength is fabricated.

In a stent according to a sixth aspect of the present invention, the main stent element has gold as a primary structural constituent and is fabricated by electroforming using an alloy of at least one selected from silver, copper, palladium, nickel or cobalt.

In a stent according to a seventh aspect of the present invention, the main stent element has palladium as a primary structural constituent and is fabricated by electroforming using an alloy of at least one selected from gold, silver, copper, nickel or cobalt.
Because an alloy capable of improving fatigue strength by greatly enhancing proof stress is selected and the stent is fabricated by electroforming, the above-described problem of the stent recited in Patent Document 2 is solved.

A stent according to an eighth aspect of the present invention is provided with a joining portion for forming the main stent element into a tubular shape.
Thus, by the stent being fabricated in a flat shape and then rolled into the tubular shape and the joining portions being joined by electric resistance welding, laser welding or the like, productivity and product quality are improved.

In a stent according to a ninth aspect of the present invention, the joining portion for forming the main stent element into the tubular shape is joined by electrodeposition.
Because an electrodeposition method is used, no heat at all is applied at the time of joining. Therefore, annealing due to heating, brittleness due to thermal deformation and the like do not occur. In addition, because the joining portion may retain the same glossiness as the raw material, the external appearance is smooth, which is advantageous when the stent is being inserted into a tubular vessel of a living body.

In a stent according to a tenth aspect of the present invention, the main stent element is formed of a plurality of cells and linking portions that link the cells to one another, a wire diameter of the cells being from 10 to 50 µm, and a wire diameter of the linking portions being from 5 to 20 µm.
Thus, because the wire diameter of the cells, that is, the wall thickness of the stent, is set from 10 to 50 µm, this is thinner than the 60 µm wall thickness of the stent recited in Patent Document 1. Thus, when the stent is placed in a blood vessel, the stent is not an impediment to blood flow and blood flow does not deteriorate. Moreover, because the wire diameter of the linking portions is narrow at 5 to 20 µm, if the blood vessel meanders in a curve, the linking portions inflect and the cells reliably support the inner wall of the blood vessel from the inner side thereof.

### ADVANTAGEOUS EFFECTS OF INVENTION

As described hereabove, according to a stent in accordance with the first aspect of the present invention, the stent is in a tubular shape that is expandable in the radial direction, being placed in a tubular vessel of a living body. The stent includes the main stent element, which is formed at an imaginary circular tube surface, and this stent element is fabricated of an alloy with a proof stress of 500 to 2700 MPa. Thus, the lower limit of proof stress of the stent element is 500 MPa, which is larger than the 300 MPa of the stent recited in Patent Document 1. Furthermore, because the upper limit is 2700 MPa, the stent may be easily and uniformly expanded in the radial direction, and may resist being easily crushed in the radial direction after expansion. Thus, these excellent effects are provided.

According to a stent in accordance with the second aspect, the stent is in a tubular shape that is expandable in the radial direction, being placed in a tubular vessel of a living body. The stent is structured by the main stent element, which is formed at an imaginary circular tube surface, and this stent element is fabricated of an alloy with a higher fatigue strength than nickel or a nickel/cobalt alloy. When an alloy is used with a strength such that this fatigue strength is at least 1.5 times higher than the strength of the nickel or nickel/cobalt alloy, as in the third aspect, this is particularly excellent for a stent in a location that is continuously subjected to vibrations, the stent may be easily and uniformly expanded in the radial direction, and the stent may resist being easily crushed in the radial direction after expansion. Thus, these excellent effects are provided.

According to a stent in accordance with the fourth aspect, because the main stent element is formed of an electroforming alloy including any among gold, silver, copper, nickel, cobalt or palladium, an excellent effect may be provided in that the main stent element may be produced by electroforming a material with a very high fatigue strength.

According to a stent in accordance with the fifth aspect, because a compound among sulfites, chlorides, ammonia complexes, cyan complexes, amine complexes, sulfamine complexes, hypophosphites, pyrophosphates, tartrates or EDTA is used as an additive, an excellent effect may be provided in that a stent with high proof stress and fatigue strength may be produced by electroforming of an alloy including any of gold, silver, copper, nickel, cobalt or palladium.

According to a stent in accordance with the sixth aspect, the main stent element is fabricated by electroforming using an alloy with gold as a primary structural constituent and at least one selected from silver, copper, palladium, nickel and cobalt. Thus, excellent effects may be reliably provided in that there is no elution at all of metal ions into any body fluid or an electrolyte, and no adverse effects at all are applied in the tubular vessel of the living body.

According to a stent in accordance with the seventh aspect, the main stent element has palladium as a primary structural constituent and is fabricated by electroforming using an alloy of at least one selected from gold, silver, copper, nickel or cobalt. Thus, excellent effects may be reliably provided in that there is no elution at all of metal ions into any body fluid or an electrolyte, and no adverse effects at all are applied in the tubular vessel of the living body.

According to a stent in accordance with the eighth aspect, the joining portion for forming the stent into a tubular shape is provided. Thus, after the stent is fabricated in a flat shape, the stent is rolled into the tubular shape and the joining portion is joined by electric resistance welding, laser welding or the like. Thus, excellent effects may be provided in that productivity and product quality may be improved.

According to a stent in accordance with the ninth aspect, because the connecting portion of the main stent element is connected by electrodeposition, no heat at all is applied during the joining. Therefore, excellent effects may be provided in that annealing due to heating, brittleness due to thermal deformation and the like do not occur, the external appearance may be made smooth because the joining portion may retain the same glossiness as the raw material, and the stent may be smoothly inserted into a tubular vessel of a living body.

According to a stent in accordance with the tenth aspect, the main stent element is structured by plural cells and linking portions that link the cells together. The wire diameter of the cells is 10 to 50 µm and the wire diameter of the linking portions is 5 to 20 µm. Thus, the wire diameter of the cells, which is to say the wall thickness of the stent, may be made thin at 10 to 50 µm, the stent does not impede blood flow when the stent is left in place in a blood vessel, and the blood flow does not deteriorate. Moreover, the wire diameter of the linking portions may be made thin at 5 to 20 µm, and when a blood vessel meanders in a curve, the inside of the blood vessel may be reliably supported in a state in which the linking portions inflect to follow the curve and the cells are in close contact with the inner wall of the blood vessel. Thus, these excellent effects may be reliably provided.

### BRIEF DESCRIPTION OF DRAWINGS

Fig. 1 is a perspective view of a stent illustrating an exemplary embodiment of the present invention.
Fig. 2 is a development view of the stent illustrating the exemplary embodiment of the invention.
Fig. 3 is a magnified view of a linking portion illustrating the exemplary embodiment of the present invention.
Fig. 4A is a view of a connection step of a main stent element illustrating the exemplary embodiment of the present invention.
Fig. 4B is a view of a connection step of the main stent element illustrating the exemplary embodiment of the present invention.
Fig. 4C is a view of a connection step of the main stent element illustrating the exemplary embodiment of the present invention.
Fig. 4D is a view of a connection step of the main stent element illustrating the exemplary embodiment of the present invention.
Fig. 5 is a graph showing the proof stress of a stent according to the present invention.
Fig. 6 is a graph showing the fatigue strength of an alloy according to the present invention.

### DESCRIPTION OF EMBODIMENTS

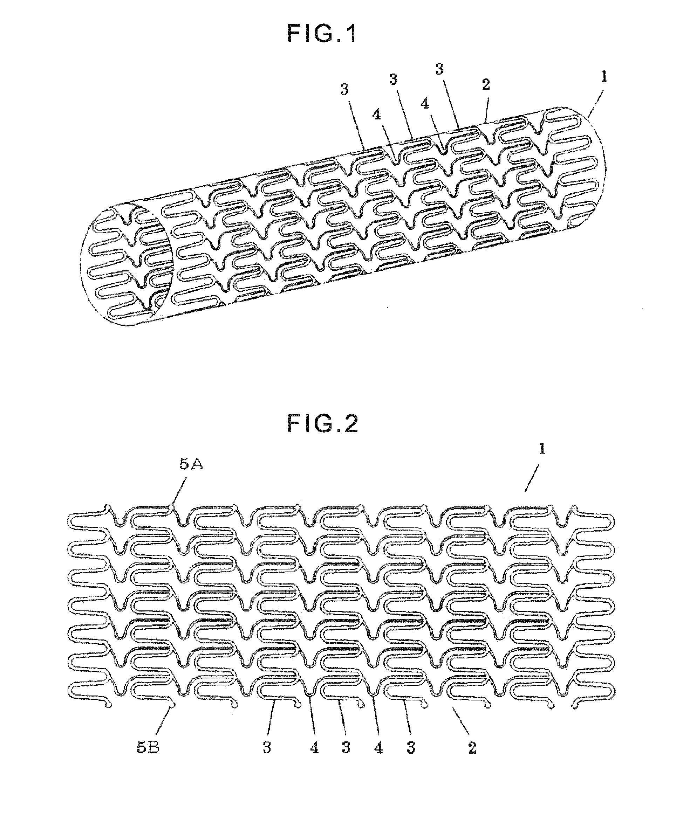

Herebelow, an exemplary embodiment of the present invention is described on the basis of Fig. 1 to Fig. 6. The present invention is not limited in any way by the following exemplary embodiment.

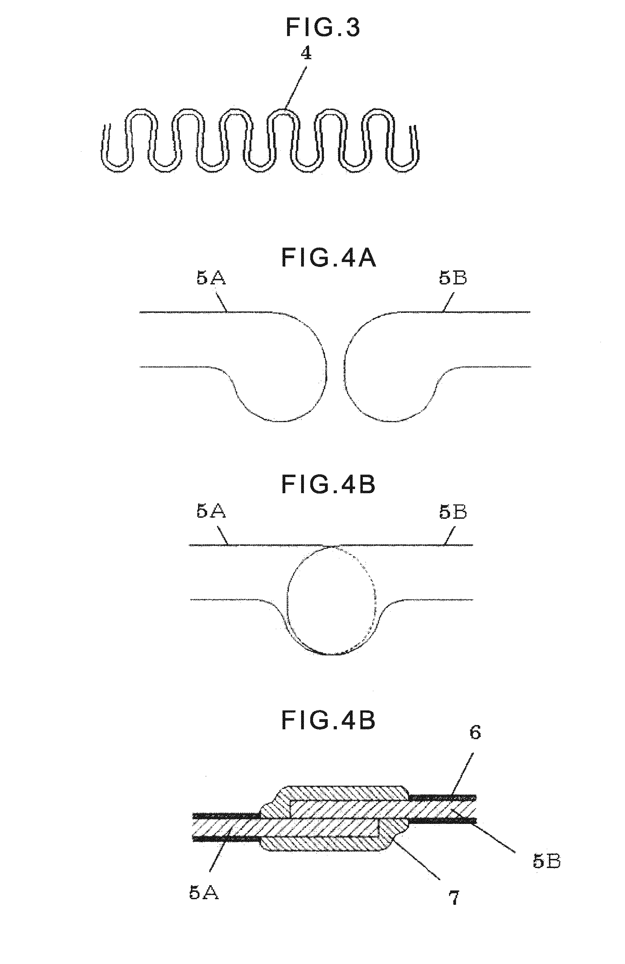

In Fig. 1 and Fig. 2, a stent 1 is a tubular stent that is expandable in a radial direction, being placed in a tubular vessel of a living body. The stent 1 includes a main stent element 2, which is formed at an imaginary cylinder surface. The main stent element 2 is formed of nine cells 3, and linking portions 4 that link the cells 3 to one another. A wire diameter of the cells 3 is from 10 to 30 µm, and a wire diameter of the linking portions 4 is from 5 to 9 µm. As shown in the magnified view in Fig. 3, each linking portion 4 is inflected in a meandering shape and has high flexibility.

In this exemplary embodiment, the nine cells 3 are separately wound. However, a single cell may be continuously wound. In such a case, the linking portions are integrally formed at one cycle intervals.
In the present exemplary embodiment, the stent 1 is produced in a flat plane as shown in Fig. 2, this is rolled into a tubular shape as shown in Fig. 1, and made into a tube by electrodeposition. However, the stent 1 may be fabricated by initially coating a resist on to a tubular mandrel and then exposing light onto a tubular shape to perform etching.

The main stent element 2 is fabricated by electroforming as described below, using an alloy with gold as a primary structural constituent and at least one selected from silver, copper, palladium, nickel and cobalt, or an alloy with palladium as a primary structural constituent and at least one selected from gold, silver, copper, nickel, and cobalt.

Firstly, a resist is coated onto a conductive support, and a photomask that blocks light is put in place. Then, ultraviolet light is exposed thereon and, depending on the characteristics of the resist, an exposed portion or an unexposed portion or the like is removed by development processing. A first cycle of deposition is performed by electroforming of the above-mentioned alloy at the removed portion, and the plural cells 3 are fabricated.
Then, the plural cells 3 are masked and, in this state, the resist is coated onto the support again and a photomask is put in place. Then, ultraviolet light is exposed thereon to remove the resist, and a second cycle of deposition is performed by electroforming of the above-mentioned alloy at the removed portion. Thus, the linking portions 4 are fabricated. Here, the exposure is performed such that portions of the cells 3 and the linking portions 4 are joined to one another. Thereafter, electrodeposition is performed, as a result of which linking bodies are formed. Thus, the plural cells 3 and linking portions 4 are integrally formed of the above-mentioned alloy, and the main stent element 2 is fabricated as shown in Fig. 2.

According to this fabrication process, the first cycle of deposition shaping and the second cycle of deposition metal shaping may be arbitrarily altered. That is, in regard to differences in width, thickness and the like, for example, the width may be dependent on a width during photomask fabrication and the thickness of a shape may be dependent on a deposition electrolysis duration. Therefore, partial regions of the stent 1 produced by the process of the present invention may be very precisely and freely altered in thickness, width and the like as necessary.
Freely and precisely controlling thickness, width and the like in this manner may provide high functionality to the stent 1 that has not been available hitherto. This is a significant feature that cannot be provided at all by a related art stent that is machined by laser or the like.

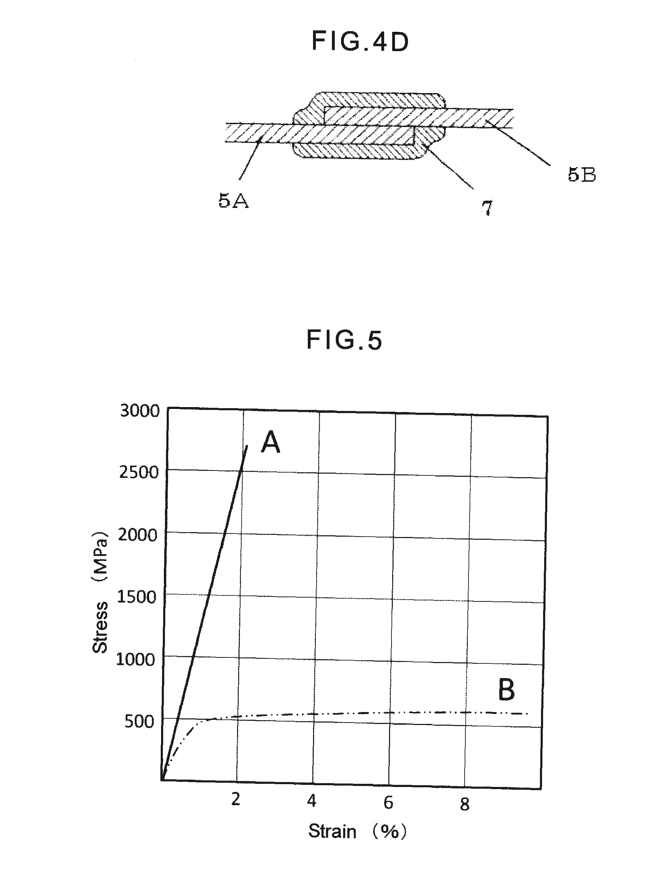

Then, the stent 1 is completed as shown in Fig. 1 by the main stent element 2 being rolled into a tubular shape and joined, by joining portions 5A and joining portions 5B of the plural cells 3 being joined to one another. The joining may be by electric resistance welding, laser welding or the like. A joining method in accordance with the joining steps in Fig. 4A to Fig. 4D is preferable. Each joining portion 5A and joining portion 5B are brought close together as shown in Fig. 4A, and overlapped as shown in Fig. 4B, after which a masking 6 of a coating, a non-conductive tape or the like is applied to portions other than the overlapped portions, as shown in the cross-sectional diagram in Fig. 4C. Then, an electroforming alloy 7 the same as the alloy of the stent 1 is added by electrodeposition, and the masking 6 is removed as shown in Fig. 4D. Thus, the joining process between the joining portions 5A and the joining portions 5B is completed.
According to this joining method, no heat at all is applied during the joining. Therefore, annealing due to heating, brittleness due to thermal deformation and the like do not occur. Moreover, because the joining portions 5A and 5B may retain the same glossiness as the raw material, the external appearance is smooth. This is advantageous when the stent 1 is being inserted into a tubular vessel such as a blood vessel or the like.

Fig. 5 is a graph showing results of measurements of the proof stress (elastic limit stress) of the stent 1. The horizontal axis shows strain (%), and the vertical axis shows stress (MPa). In Fig. 5, A shows a case in which an alloy of 40% nickel and 60% palladium was used as the alloy of the main stent element 2, and B shows a case in which a conventional alloy of 80% nickel and 20% cobalt was used as the alloy. Thus, it could be confirmed that the proof stress of the stent 1 according to the present invention was much larger, at the 2700 MPa of case A, than the 500 MPa of case B.

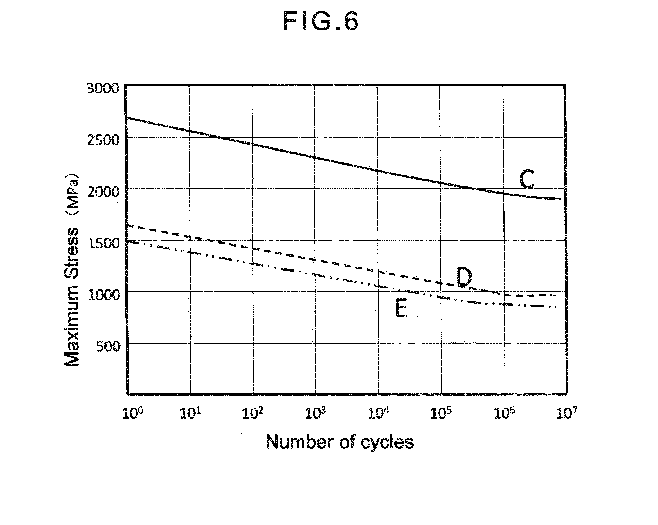

Fig. 6 is a graph showing results of measurements of fatigue strength. The horizontal axis shows a number of repetitions (cycles) and the vertical axis shows maximum stress (MPa). In Fig. 6, C shows a case in which an alloy of 40% nickel and 60% palladium was used as the alloy of the main stent element 2 in accordance with the present invention, and D and E show cases in which conventional materials were used, case D being a nickel/cobalt alloy and case E being nickel. When the maximum stress was 1000 MPa, case D broke after 700,000 cycles and case E broke after 50,000 cycles. In contrast, case C according to the present invention had not broken even after 10 million cycles. Thus, it could be confirmed that the fatigue strength was larger, 1.5 to 14 or more times greater than that of a related art nickel/cobalt alloy or nickel.

Metals that are electroformable are limited. A typical example of a metal that may be plated but may not be electroformed is chromium. This is due to the fact that chromium is not stable because of powerful stresses that occur in electrolysis, which are caused by metal ions or metal oxide ions. There are also metals with which even electroplating is difficult; for example, titanium, aluminium, molybdenum, tungsten and so forth. This is because it may not be possible for a single metal that oxidizes strongly, such as these, to exist as ions in an aqueous solution. When electroplating alloys too, stresses during electrolysis are often a problem. Recent technologies are making the realization of low stresses in electrolytes possible, by the introduction of various water-soluble metal compounds.

To control the composition of an alloy, it is necessary to bring respective metal deposition potentials of the metals that are being alloyed close together. For this, it is necessary to utilize various complex and chelate compounds rather than just simple salt compounds. In production by electroforming of the alloy according to the present invention, which is an alloy of two or more of the six metals gold, silver, copper, nickel, cobalt or palladium, the stent 1 is fabricated with a high proof stress and fatigue strength by using supporting electrolyte compounds, which are inorganic and organic additives, such as sulfites, chlorides, ammonia complexes, cyan complexes, amine complexes, sulfamine complexes, hypophosphites, pyrophosphates, tartrates, EDTA (ethylenediaminetetraacetic acid) and the like.

Next, to check the corrosion resistance of an alloy, the alloy was submerged in a sterilizing fluid such as physiological saline, a solution with a pH from 3.0 to 8.0, Milton (registered trademark) or the like. Then, the sample was forcibly subjected to vibrations at 100 kHz for accelerated elution testing in a test solution, after which a metal ion elution test was conducted. The solution was analyzed by atomic absorption spectroscopy; the analysis limit was parts per billion (PPB).

From the results, the compositions that did not elute metal ions were the following.
1) An alloy of gold and Palladium: No metal ions at all are eluted, whatever the composition.
2) An alloy of gold and any of silver, nickel and cobalt: No metal ions at all are eluted if the gold content is 65% or more.
3) An alloy of palladium and any of gold, silver, copper, nickel and cobalt. No metal ions at all are eluted if the palladium content is 60% or more.

It was confirmed that if the main stent element 2 is fabricated by electroforming an alloy, using an alloy selected from an alloy of 30% gold and 70% palladium, an alloy of 90% gold and 10% silver, an alloy of 35% cobalt and 65% palladium, an alloy of 80% gold, 15% silver and 5% cobalt, and an alloy of 80% gold, 15% palladium and 5% cobalt, an effect of proof stress (elastic limit stress) and fatigue strength being similar to those described above is obtained.
Any stent 1 fabricated with these compositions exhibited excellent X-ray absorption, excellent practical performance and acceptable performance in regard to corrosion resistance, equivalent to or better than stents fabricated with conventional materials such as stainless steel, cobalt, chromium and the like.
The stent 1 of the present invention is formed of a homogeneous composition and is not magnetic. Thus, artefacting in images from diagnostic instruments that use strong magnetic fields such as magnetic resonance imaging (MRI), which has become widespread in recent years, is excellent compared to related art items.

In recent years, the following measures have been applied to stents with a view to preventing restenosis of the tissue of a tubular vessel, such as a blood vessel, the alimentary canal or the like, after a stent is inserted.
1) Coating metal surfaces of the stent with a composition that improves relative antithrombocity, such as pyrolytic carbon (PC), diamond-like carbon (DLC) or the like.
2) Coating metal surfaces of the stent with a polymer and treating the surface of the polymer with an antithrombotic material.
3) Coating metal surfaces of the stent with a polymer, then introducing a drug that suppresses tissue growth into the polymer, and allowing controlled release of this drug from the polymer.
4) Coating metal surfaces of the stent with a biodegradable polymer, then introducing a drug that suppresses tissue growth into the polymer, allowing the polymer to gradually degrade, and completely releasing the drug over a certain period.
5) Forming small dimples (irregularities) in the surface of the stent, introducing a drug into the dimples, and providing a tissue growth prevention effect for the duration of a relatively short period.
These measures may be easily applied with the stent 1 of the present invention.

In particular, for a stent that is applied to a tubular organ of the digestive system, the respiratory system or the urinary system, hypertrophy of cancer cell tissues, restenosis of the tubular organ or the like should be prevented. Therefore, a stent formed of a main stent element for expanding the tubular organ and an auxiliary stent element with a higher stent cell density for suppressing extension of the tissues, or the like, is made. In the present invention, because a material with excellent strength may be provided, items that are excellent in fine structure and strength may be easily realized.

Nowadays, particular designs are used, such as the following.
1) A stent to be placed in a branching blood vessel (a Y-stent). This assures blood flow through the branched blood vessels, and the cells of a tubular portion of the stent are widely opened in order to facilitate the approach of another device such as a catheter or stent.
2) Alternatively, to make a stent with which a stent longitudinal direction rigidity can be adjusted and a direction of curvature is flexible, with a structure that is easy to insert, stent longitudinal direction stent linking elements of auxiliary stent elements and suchlike are made narrower than a main stent element.
The present invention may be easily applied to any of these designs.

### - Operation -

The present exemplary embodiment is structured as described above, and operations thereof are described below. In a state in which a balloon of a balloon catheter is deflated, the stent 1 shown in Fig. 1 is mounted at the outer side of the balloon in a reduced-diameter state. Then the stent 1 is inserted into a blood vessel together with the balloon, the balloon is disposed at a constriction portion in the blood vessel, the balloon is inflated, and the stent 1 is inflated and expanded. The stent 1 is left in place, retaining its expanded state, and the balloon catheter alone is taken out.

As a result of tests of this operation using test materials corresponding to blood vessels and blood, it has been confirmed that, because the proof stress of the main stent element 2 is from 500 to 2700 MPa, the stent 1 is easily and uniformly expanded in the radial direction, and is not easily crushed in the radial direction after expansion.
Furthermore, it has been confirmed that, because the wire diameter of the cells of the main stent element 2 is from 10 to 30 µm and the wire diameter of the linking portions is from 5 to 9 µm, the stent 1 is not an impediment to blood flow even when the stent 1 is placed in a blood vessel, and blood flow does not deteriorate. It has also been confirmed that, if a blood vessel meanders in a curve, in a state in which the narrow linking portions inflect to follow the meander and the cells 2 are in close contact with the inner wall of the blood vessel, the blood vessel is reliably supported from the inner side thereof.

Explanation of Reference Numerals

| | |
|---|---|
| 1 | Stent |
| 2 | Main stent element |
| 3 | Cell |
| 4 | Linking portion |

## Claims

1. A tubular stent that is expandable in a radial direction, the stent being placed in a tubular vessel of a living body and comprising a main stent element that is formed at an imaginary cylinder surface, the main stent element being fabricated of an alloy with a proof stress of from 500 to 2700 MPa.

2. A tubular stent that is expandable in a radial direction, the stent being placed in a tubular vessel of a living body and comprising a main stent element that is formed at an imaginary cylinder surface, the main stent element being fabricated of an alloy with a higher fatigue strength than nickel and nickel/cobalt alloys.

3. The stent according to claim 2, wherein the fatigue strength of the alloy of the main stent element is at least 1.5 times the strength of nickel and nickel/cobalt alloys.

4. The stent according to any one of claims 1 to 3, wherein the main stent element comprises an electroformed alloy including one of gold, silver, copper, nickel, cobalt or palladium.

5. The stent according to claim 4, wherein the main stent element is fabricated by electroforming using the alloy including one of gold, silver, copper, nickel, cobalt or palladium, to which is added a compound selected from sulfites, chlorides, ammonia complexes, cyan complexes, amine complexes, sulfamine complexes, hypophosphites, pyrophosphates, tartrates or EDTA.

6. The stent according to claim 4 or claim 5, wherein the main stent element has gold as a primary structural constituent and is fabricated by electroforming using an alloy of at least one selected from silver, copper, nickel, cobalt or palladium.

7. The stent according to claim 4 or claim 5, wherein the main stent element has palladium as a primary structural constituent and is fabricated by electroforming using an alloy of at least one selected from gold, silver, copper, nickel or cobalt.

8. The stent according to any one of claims 1 to 7, wherein the main stent element includes a joining portion for forming the main stent element into a tubular shape.

9. The stent according to claim 8, wherein the joining portion of the main stent element is joined by electrodeposition.

10. The stent according to any one of claims 1 to 9, wherein the main stent element is formed of a plurality of cells and linking portions that link the cells to one another, a wire diameter of the cells being from 10 to 50 µm, and a wire diameter of the linking portions being from 5 to 20 µm.
